# EUROPEAN PATENT APPLICATION

(11) **EP 2 647 351 A1**
(43) Date of publication of application: **09.10.2013**
(21) Application number: 11845231.7
(22) Date of filing: 03.10.2011
(51) Int. Cl.: A61F 2/04, A61L 27/38

(54) **FLAT DEVICE FOR FACILITATING THE TRANSPLANT OF BIOLOGICAL MATERIAL**

(30) Priority: 30.11.2010 MX 2010013135
(71) Applicant: Val De Bio, S. De R.L. De Cv., Estado de México (MX)
(72) Inventor: ORTIZ-AUSTIN, Annette Gisela, 04370 Mexico D.F. (MX); VALDES GONZALEZ-SALAS,Rafael Alejandro, 76230 Juriquilla Queretaro (MX)
(74) Representative: Karcz, Katarzyna
(86) International application number: PCT/MX2011/000116
(87) International publication number: WO 2012/074349

(57) **Abstract**

The present invention describes a flat or improved flattened device to favor the implant of cells, that once it has been inserted in the subcutaneous tissue the device is coated by fibrocollagen, thus generating an immunologically privileged site inside the device, site which is useful as neovascularized reservoir for the implant of cells producers of biological factors, for the treatment of diseases such as diabetes mellitus, SNC or for the implant of primed cells against tumors.

## Description

### Technical Field of the Invention

This invention is related to the field of biomedicine, biotechnology, biomaterials and other subjects related to the use of medical devices particularly with respect to cells implants for the production of biological factors for the treatment of chronic degenerative diseases or diseases caused by any deficiency of biological factors. More specifically, this invention is related to a device generator of an immunologically privileged site for the implant of any kind of cell line either *in vitro* or *in vivo.*

### Background of the Invention

The deficiency of a biological factor in an individual is the main cause of appearance of chronic degenerative diseases like diabetes mellitus, Parkinson's disease, hypothyroidism, hormonal disease and others.

The traditional treatments of some of these diseases have consisted in the application administration of the deficient biological factors individuals or substances that stimulate their production, generally by means of injections of products obtained from chemical synthesis or by biotechnology. This type of treatment shows several disadvantages especially related to the frequency of doses required to maintain the factor at an optimum level, which is practically impossible to achieve. This nevertheless is still the method that is most frequently used, since it is the easiest and cheapest option to maintain the factor at the required limits.

In order to enhance the bioavailability of the factor, attempts have been conducted to develop methods, devices and apparatus to control its release.

An alternative refers to pumps to control the dosage of the biological factor based on the required or demanded dose, fact which has been simplified with the use of these apparatuses, their cost makes them unaffordable for all the population. In addition, a commitment from the user is required in order to optimize its operation.

Another alternative that has been tried is the implant of cells that produce the biological factor. The direct contact of cells with the body of the receptor however causes a implant rejection reaction which is evident by means of the formation of tissues that prevents the flow of nutrients with the consequent destruction of cells so that the life of the implanted cells is relatively short and the transfer of biological factors is limited. Consequently their therapeutic effect is deficient. The tissues that appear to reject the implants are constituted by cells called lymphocytes, plasmatic cells and antibodies. Fibrocollagen is the means to cover foreign bodies even when those bodies are positives.

In order to try to avoid the problems of implant rejection derived from the direct implant of cells, a variety of devices has been designed that generally consist of a chamber or capsule where the cells are placed, in such a way that these cells are isolated and do not have contact with the individual's immune system thus preventing the formation of antibodies. The devices for implant that contain the cells are generally made of natural polymers like collagen and alginates or synthetic polymers such as polyacrylates, vinyl acrylonitrile, and poly-xylene.

In US Patent No 5'614,205 (Usala) for instance, a matrix is described consisting of a poly-para-xylene membrane and a cell culture that produces insulin for the treatment of diabetes mellitus. The membrane has certain porosity that allows the passage of nutrients and biological factors but prevents the passage of immune agents. The patent mentions that the biocompatible material does not produce rejection.

US Patent No 5,569,462 (Martison et al.) describes that the mortality of the cells producing the biological factor of interest occurs due to the fact that the flow of nutrients and waste products are not adequate during the ischemic period of the implant. The alternative al consists of the use of a device with a chamber for cells, where said chamber is immuno-isolated with biocompatible material such as polytetrafluoroethylene (PTFE) 15-micron in width and 5-micron porosity. Additionally the uses of immuno-modulatory agents such as immunosuppressive agents like mycophenolic acid, cyclosporine, rapamacyn, etc., or like anti- inflammatory agents such as corticosteroids are required. Furthermore, it is well known that the use of products to suppress the immune response and to inhibit the recognition and rejection of transplants and/or implants such as cyclosporine has negative effects on neovascularization, so that increases the probability of an unsuccessful transplant or implant. The above mentioned devices do not solve satisfactorily the various disadvantages of implants, because despite these materials are biocompatible, there is still tissues are formation and inadequate vascularization around the device in relatively short periods of time after the implant, so that the bloodstream supplied to the tissues in that region is very low and therefore the availability of nutrients is also low.

The construction materials of the devices despite being permeable, constitute an additional barrier for the exchange of nutrients and biological factors between the implanted cells and the patient body. The US patent No 5'725,854 (Selawry) claims a method for the treatment of diseases that comprises the administration of Sertoli cells together with cells that produce the biological factor an attempt is made to create an immunologically privileged site. It is well known that the Sertoli cells promote the immunological tolerance and contain a high amount of elements for protecting the cells responsible for the production of biological factor and to maintain their functioning for an indefinite period of time However since this alternative does not suppress totally the rejection, it is therefore necessary to continue the administration of immunosuppressive or immunomodulatory drugs, which in turn has negative effect on the neovascularization.

A better alternative is the device referred in the US Patent No 6,716,246 (Valdés). *Process and device for facilitating the implant of biological material,* which describes a device that use fibrocollagen produced by the receiving body, in order to create an immunologically privileged site for receiving biological factor producing cells or primed cells for the treatment of malignant diseases (WO2009/075556 (Valdés). (Procedure for priming cells and their use for the treatment of tumors). The density of the fibrocollagen produced by this device is relatively high, so that the device can be maintained isolated thus forming a thick layer of fibrocollagen composed of neoformed vessels, thus creating a natural reservoir to generate immunological tolerance.

A variant of the device referred in the US patent No 6,716,246 (Valdés) is the denominated *Cell Pouch System*™ of *Sernova Corporation* that proposes the same circular device but instead of having only one tube, they propose 8 tubes horizontally aligned one next to the other hold with a band in one end. This device however still has the same problems with the circular devices.

On the other hand, in the last years the xenoimplantation has gained importance for clinical use, examples of this are the experiments that were successful to implant neuronal cells of fetal pig in the brains of 24 patients affected by Parkinson's or Huntington diseases (Fetal pig neuronal cells for Parkinson's Disease, Diacrin, Inc. started in April 1995 the phase I clinical trials. The initial clinical center was Lahey Hitchcock Clinic, Mass.) The purpose was to attain that the implanted cells would behave in a similar way to the brain implants of human fetal cells (http://www.genvec.com/). On the other side scientific experience has been developed for the implantation of porcine adrenals cells in the spinal cord of 36 terminal cancer patients with intractable pain, with the purpose of the implanted cells to produce specific substances capable of blocking the pain signals (fetal adrenals cells (encapsulated) implanted in the space of the spinal cord to relief pain in terminal cancer, Cytotherapeutics, Rhode Island). The success of both treatments was partial but very promising.

In the same way, a scientific group encapsulated porcine pancreatic cells with the purpose of stimulating the production of insulin in 50 diabetic patients by trying to avoid the patient immune response that eventually would destroy the implanted cells. (Porcine pancreatic islet (encapsulated) implanted for the treatment of insulin-dependent diabetes mellitus VivoRx, Minnesota and California). The short term result was fairly good and it was not successful in the long term.

Nowadays a considerable number of scientific groups have the purpose of using hepatic cells, genetically modified pig livers (The genetically modified pig liver is used for ex vivo perfusion for the treatment of fulminant hepatic failure, Dr. Platt, Duke University, North Carolina) or bioartificial livers composed of porcine hepatocytes (HepatAssist System 2000) for treating patients with hepatic failure. An example of this are the experiments conducted in which the blood of 54 patients was passed extracorporeally through bioartificial livers of porcine hepatocytes, used as temporary bridges to keep them alive until receiving the implant or until recovering their hepatic function (Pless G, Sauer IM. Bioartificial liver: current status. Implant Proc. 2005 Nov:37(9):3893-5). Dr. Suzanne IIdstad, from the University of Allesheny achieved scientific relevance when she implanted bone marrow from a baboon monkey to a patient suffering from AIDS; the patient remained alive during 13 days (Michaels MG, Kaufman C, Volberding PA, Gupta P, Switzer WM, Heneine W, Sandstrom P, Kaplan L, Swift P, Damon L, IIdstad ST. Baboon bone-marrow xenoimplantat in a patient with advanced HIV disease: case report and 8- year follow-up. Implantation 2004 Dec 15;78(11):1582-9.) (Baboon bone marrow implant for HIV, Dr. IIdstand, UCSF General Hospital, single-patient IND, and FDA allowed IND to proceed August 1995*. Implant took place Dec. 14, 1995. The absence of evidence of baboon cell engraftment was reported February 1996.)*

One of the most promising results of the xenoimplantation use for the treatment of diseases is the one achieved by the group of Dr. Rafael Valdés (Valdés-González RA, Dorantes LM, Garibay GN, Bracho-Blanchet E, Mendez AJ, Dávila-Pérez R, Elliot RB, Téran L, White DJ. Xenoimplantation of porcine neonatal islets of Langerhans and Sertoli cells: a 4-year study. Eur J Endocrinol. 2005 Sep;153(3):419-27) who could maintain a group of patients diagnosed with type I *Diabetes mellitus* under control without the administration of insulin for a period longer than 10 years, by using the implant of porcine pancreatic cells in a device to facilitate the implant of cells (US Patent No US 6,716,246 Valdés). Results showed that the survival of the implanted cells was attained with the consequent production of insulin.

The clinical future of implants and particularly of the xenoimplants is very promising for concrete applications with cells and tissues for the treatment of complex diseases deficient of a biological factor and it is being assessed as a complementary method for the treatment of cancer.

Although promising results have been obtained with the device referred in the US patent No 6,716,246 (Valdés), this device still has some deficiencies. Circular devices have the inconvenience that the contact area between the new fibrocollagen tissue and the biological material implanted for the desired production of biological factors is limited. A possible solution to the limited contact area in the circular devices is the use of multiple devices like that denominated *Cell Pouch System*™ of *Sernova Corporation.* However, each of these cylindrical elements must be filled with the biological material to be implanted and this process does not warrant homogeneity among cylinders nor the cell survival. On the other hand, according to the provided description of the circular devices, the implant of the biological material producer of the desired biological factors leaves a dead space because only the implanted cells that are in contact with the neovascularized fibrocollagen survive in the short time, fact which limits the function of the implanted device. So, the circular devices composed either of a single or by several cylindrical structures have the inconvenience that the cell viability and particularly the dead space generated at the center of the device cannot be accurately determined. It is a factor of death because cells are left conglomerated in the inner chamber bringing about that a large number of implanted cells do not survive because they do not receive nutrients. Besides the number of surviving implanted cells cannot be quantified or controlled.

Other inconvenience of the referred devices deals with the method of filling of the biological material to be implanted into the device. The cells to be implanted are suspended in an aqueous medium and when the medium is introduced in the orifices either into one or more circular tubes of the device it is inevitable the death of certain amount of cells to be implanted. In addition, the number of cells that will to adhere to the surface of the neovascularized tissue is uncertain.

One of the objectives of this invention therefore, is to provide an improved device, generator of immunologically privileged sites, that can be used for receiving the implant of cells producer of biological factors for the treatment of diseases, like the implant of cells for the treatment of diseases like Parkinson, CNS and cancer. This device should minimize to the utmost the inconveniences that circular devices have particularly that related with the dead space created in the interior and the lack of control on the viability of the implanted cells.

Another objective of the present invention is to provide an immunologically isolated site with particular characteristics to allow a good neovascularization for the adequate transfer of nutrients and biological factors in such a way that allows a larger number of cells to have contact with the blood vessels therefore favoring the survival of a larger number of cells and as consequence, a better performance for the production of biological factors.

Another purpose of this invention is to provide a safer and more effective method for the implant of biological material that allows a more certain quantification of the implanted material and also that allows a higher survival of the material after the implant thus maintaining the functionality of the device in the long term.

This and other purposes will be described with more details in the next detailed description of the invention.

### Brief description of the drawings

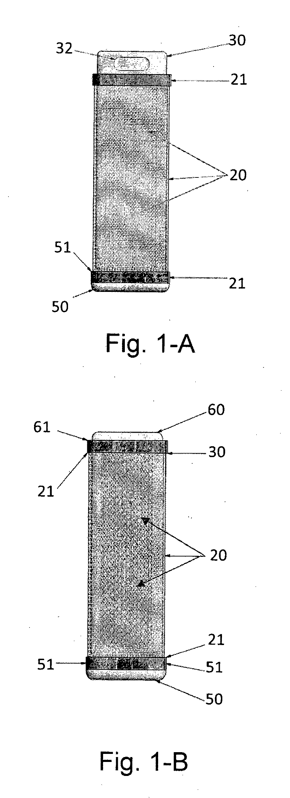

Figure 1 represents a general view of the device where in part 1-A, it can be seen the device with the plunger inserted (30). The device has in one end (31) a wedge (32) that facilitates the plunger withdrawal when the fibrocollagen is already formed. Part 1-B represents a general view of the device with the cell culture tray (60) inserted.

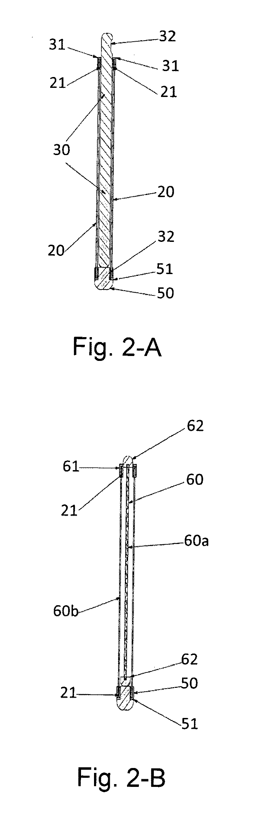

Figure 2 represents a sagital section of the device over its midline. Part 2-A shows with more detail the plunger (30) inserted in the body of the device (20), with a wedge in one end of the device (32). Part 2-B. shows the body of the device (20) with the cell culture tray (60) inserted. As it can be seen there is a minimum space around its two sides, fact which allows the contact of all the implanted cells with the neoformed vessels of the fibrocollagen.

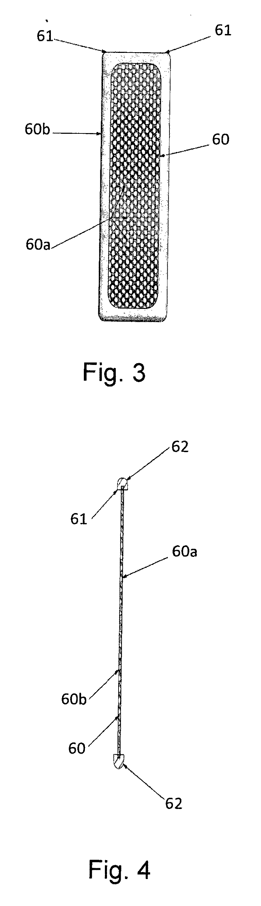

Figure 3 shows a front view of the tray, which shows its gridded base that allows the adhesion of the implanted cells.
Figure 4 corresponds to a side view of the cell culture tray (60), that shows in the ends, the abutments/supports (61) which function is such that when the tray is inserted in the body (20), it leaves a space enough so that the cells fixed to both sides of the cell culture tray culture (60), be in contact directly with the neoformation vessels.

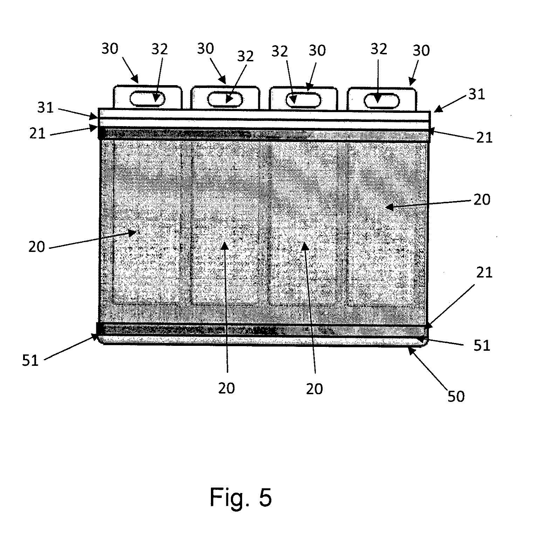

Figure 5 shows a preferred embodiment in the present invention with different plungers (30) inserted, these plungers will be exchanged by culture trays. The advantage is that with only one incision, various cell culture trays can be inserted.

### Detailed description of the invention

The present invention refers to an improved device which is inserted in the subcutaneous cell tissue, in order to facilitate the implant of cells. This device differs from those devices currently known because it is flat shaped, form which decreases the dead space in it inner chamber, as it occurs with the circular devices, thus allowing an extensive contact of the implanted cells with the neovascularized surface in such a way that cells in liquid media can also be implanted directly. In addition, it comprises a novel tray whose design and shape, allows the cells can be seeded in both anterior and posterior sides, thus increasing even more the useful space so that every cell to be implanted, will be seeded in such a way that will be adhered to the sides and will contact immediately the neoformation vessels, thus allowing the cells to interact with the media so that there is no lack of nutrients thus increasing the cell survival rate and therefore the device functionality. This novel flat or flattened device, with its culture tray, allows the cells to implant to be seeded in vitro in both sides of the tray before being inserted in the body of the device covered by fibrocollagen. This fact allows to know accurately the amount of seeded cells and to determine their viability before being inserted. In addition, in a preferred embodiment and as a variation of this invention, the device is composed of several plungers resembling a comb, one next to the other, held in one of its ends, that when the plungers are replaced by the cell culture trays it facilitates the implanting procedure. The number of culture trays depends on the number of cells to be implanted. Although it could be thought that the use of a widest culture tray would be simpler, this invention has proved that the device described in the present invention allows a better control of the number of cells to be implanted, by handling the cells in independent trays in the comb arrangement. In addition another important aspect is that the dimensional integrity of the porous body is maintained.

With the present invention any kind of cell line can be cultured for the treatment of chronic degenerative diseases, diseases generated by the deficiency of a biological factor or for the treatment of malignant tumors, among other diseases.

In accordance with Figure 1, the device object of the present invention consists of an improved device which contains one hollow section or body (20) preferably with a porous surface, and has a cavity inside it that houses a plunger (30). At the ends of the porous body (20) it can be found sealing sets or mechanisms (21) one of which connects to the one sealing element (50) while the other end (21) connects to the second sealing element (31) one end of which is joined to the plunger or to a second sealing element (61) which is integrated to the cell culture tray (60), in such a way that, when the device is closed by means of the sealing plugs (31) or (61) it keeps rigidly inside the porous body (20) said plunger (30) or the cell culture tray (60) as can be seen with more detailed in Figure 2.

The porous body (20) is preferably composed of a rectangular or square grid with rounded corners and edges that can be made of biocompatible stainless steel, biodegradable inert polymer or any other material capable of providing with dimensional stability to the intermediate part of the set of the device thus allowing the contact of the neoformed blood vessels with the implanted cells by means of the cell culture tray (60). In accordance with the purposes of the present invention, the degree of porosity of the device porous body (20) in order to achieve the goals the present invention must have a mesh size of 40 to 150-mesh or higher if the implant of primed antitumor cells is required. On other side, the length of this intermediate porous section or porous body (20) can be the adjusted according with therapeutic needs in order to favor appropriately the production of the biological factor needed. The preferred length is 10 to 600 millimeters with an inner space preferably between 0.1 mm and 3 mm. The device has two ends having each the sealing elements (21). One of this is coupled to the plunger (30) sealing element (31) or to the cell culture tray (60) sealing element (61) in such a way that, when the sealing elements are closed, the inner cavity of the porous body (20) becomes sealed. In its other end, the sealing element (21) is coupled to the plug (50) sealing element (51) plug which has a shape and design that when coupled to the porous body (20) sealing element (21) seals the end and gives continuity to the mesh in the bottom of the porous body. If it is used under the modality to receive the comb plunger, the porous body (20) is divided into two or more sections, although 4 are preferred, which are attached to the arms of the comb-shaped plunger. Figure 5.

The plunger (30) has a size, shape and design such that it can be introduced in the cavity of the porous body (20) and since it has a sealing element (31) in the end which has the wedge (32), the plunger allows the inner of the porous body to be sealed (20) aimed at preventing the contamination of the inner cavity by adventitious agents. In the exterior part of its end the plunger has a wedge (32) whose main function is to hold firmly the plunger to the porous body when withdrawing the plunger from the porous body so as to withdraw with higher strength. The plunger (30) is made of any biomaterial which can be introduced into the organism of a mammal, without being rejected but that at the same time it should produce an important response from the organism to a foreign body in order to promote the coating with fibrocollagen. Teflon is the material preferred for this invention The details of the plunger (30) can be seen with more detail in Figure 2A or in Figure 5, if the device corresponds to the comb-shaped one.

The culture tray (60) has a design and shape such that it can be introduced into the porous body (20). Its sealing element (61) when coupled to the sealing element of the porous body seals the inner cavity thus promoting the growth of the seeded and implanted cells. Its anterior (60a) and posterior (60b) sides are made of stainless steel medical degree mesh or of any other material accepted for implant with such porosity that allows the cells to be seeded to adhere to both surfaces of the anterior (60a) and posterior (60b) sides so that in a subsequent step, to be inserted into the body (20) of the device. In each of its edges, the culture tray (60) has an abutment/support (62) with shape and dimensions such that the tray becomes adapted to the inner space of the porous body. The function of such abutment/support is to keep fix the cell culture tray within the porous body (20) and when the tray is fixed in this position it provides with the necessary space between the sides (60a and 60b) of the culture tray (60) and the inner anterior (20a) and posterior (20b) sides of the porous body (20), in order to favor the immediate contact between the seeded cells in the culture tray (60) and implanted into the inner chamber of the porous body (20) covered by fibrocollagen and the neoformed vessels. In a preferred embodiment of this invention, this space is 0.1 mm. to 3mm. Figures 3 and 4 show more details of the culture tray (60).

In a preferred embodiment of the present invention various culture trays (60) are used one next to the other as it can be seen in Figure 5. The number of culture trays will be the one required to implant the number of cells required by the by the receiving organism. Several cell culture trays (60) may be used instead of only one, wider, in order to avoid that the wider porous body (20) collapses.

In accordance with the procedure of the present invention, the improved device for the implant of cells, once that is introduced within the subcutaneous tissue of any mammal, the first sealing element (31) that is fitted to the plunger (30) coupled to the sealing element (21) of the porous body (20), will produce a foreign body reaction thus bringing about the device to be coated by fibrocollagen of the mammal in which the device was implanted. Once the plunger (30) is withdrawn withdrawal which is facilitated by its wedge (32), produces an inner site immunologically isolated or privileged, which is appropriate to house the tray containing the cell culture (60) either previously cultured or *in vitro.* The novel tray for cell culture (60) has design such that cells can be cultured on both sides and that thanks to its abutment/support (62) allows that the cell culture tray (60) remains fixed in the inner chamber of the porous body (20) of the device thus leaving an inner space within the chamber enough for the seeded cells in one or both anterior and posterior sides (60a) (60b) to contact immediately the neoformed vessels of the fibrocollagen. In a preferred embodiment of the present invention, this space ranges between 0.1 and 3 mm.

It has been determined that the degree of porosity of the grid composing the intermediate porous body (20), in accordance with the procedure herein explained and also with the functions of the patient's organism, is determinant of the size of the neoformed vessels in the fibrocollagen. Therefore the size of the mesh or pore is determined according to the type of application to be given to the tube of fibrocollagen recently formed.

In accordance with one of the preferred modalities the plunger (30) or plug (50) sealing elements (31) or (51) respectively, consist preferentially of a press-activated sealing element with a latch as fastening element that generates a hook click so that the user can ensure that it has been sealed. The sealing elements are inserted in one of the sealing elements of the porous body (21), thus holding the porous body to the sealing element (31) or (51).

The applicant has found that the thickness of the of the device porous body (20) of the porosity, is related to the size of the neoformed vessels. Therefore porosity is defined in accordance with the most appropriate conditions for the survival and growth of the cells to be implanted in maintain an effective therapeutic action.

The device is manufactured with medical degree biocompatible materials. These materials for instance, can be made of some kind of stainless steel, of virgin polytetrafluoroethylene (PTFE), titanium, biodegradable polymers, biopolymers, etc.

As it can be inferred from the description of the flat or improved flattened device for favoring the implant of biological material, its composing parts can be made by means of machining or by means of mold injection. The chosen process will be determined depending on the composing materials to be used.

In accordance with the present invention, the procedure for the implant of biological materials under the modality of reservoir, product of the formation of the biologic fibrocollagen tube by using the already mentioned device, consists of the implantation in the body of any specie of mammal including humans, the device together with the plunger (30) inserted in the inner chamber of the porous body (20), in such a way that, when the device is implanted, the mammal organism naturally coats the porous body (20) with fibrocollagen. Then once that the layer of fibrocollagen is formed, a partial incision is made, with the purpose of exposing the part of the device that has the sealing element (31) coupled to the sealing element (21) of the porous body (20), with the purpose of withdrawing the plunger by facilitating this process by means of the wedge (32). Once the plunger (30) has been withdrawn there is a chamber made of neovascularized fibrocollagen appropriate for the implant of cells seeded on the culture tray (60). The tray can then be inserted through the opening appearing when the plunger or flat tablet is removed (30). The space remains sealed due to the coupling of the sealing elements of the porous body (21) and of the culture tray (61). In these terms, the cells producer of the biological factor, start producing when entering in contact with the neoformed and vascularized collagen fiber tissues thus being the biological factor absorbed by the blood stream. In another modality, if the device was chosen with several trays of cell culture (60), the same procedure is followed but only the necessary trays of cell culture are inserted (60).

In order to increase the effectiveness of the treatment, cells genetically manipulated by known techniques can be used to produce the biological factor.

The culture medium to be used is selected in terms of the cells to be implanted. With the aid of cytoprotective agents like niacin or allopurinol The culture medium can be placed directly in the immunologically privileged space generated by the device, without the need of the tray Thanks to the flatted-shape of the device, the implanted cells are not compressed.

The applicant has found that with the use of the device described in the present invention, it has been possible to obtain semi-isolated sites with good appropriate neovascularization and therefore, conditions exist for a better cell viability of cells. Similarly, a better interchange rate of biological factors is obtained in, comparison with the circular devices.

The amount of cells, for the case of the treatment of diabetes referred in the literature is about 6,000 to 12,000 Islets of Langerhans per kilogram of the patient's weight. In the case of the present invention, it has been seen that these can be combined or not with Sertoli cells in order to immunologically protect them from the rejection. These devices can also be used for the implant of any cellular culture required for the treatment of different diseases. This includes primed cells against tumors, since it has been confirmed that the implanted cells in the device in the US patent No 6,716,246 migrated from the device without being rejected by the recipient organism fact that has opened the possibility for this kind of treatments as referred in the patent application No W02009/075556. Procedure to prime cells and its use for the treatment of tumors.

### Examples

Under the preferred embodiment of favoring the implant of cells producers of a biological factor, the flat or flattened improved device object of the present invention was implanted in the dorsal part of a sample of Long Evans rats weighing between 180 and 200 grams. In a second group of rats with the same characteristics the circular device referred in the US patent No 6,716,246 (Valdés) "Process and device to facilitate implantation of biological material" was implanted. Diabetes was induced by means of an intravenous application of 65 mg/kg of streptozotocin to the groups of ten rats with the device object of this invention and to a control group. The level of glucose in both groups showed no important differences, being in the order of 337mg/dL.

An implant of islets of Langerhans was performed with both groups and the islets of Langerhans were isolated and conserved using known conventional techniques, with the difference that the group of rats with the flat or flattened improved device received the Islets of Langerhans in the site formed by the cell culture tray culture (60). Whereas the group in which the circular device was implanted, the cells were implanted, directly in the formed fibrocollagen tubes. Both groups were implanted with the same number of islets, which was in the order of 50 to 100 thousand IEQs. Glucose levels were measured daily during the first week and subsequently once a week. The animals with the circular device group showed in the first three days a glycemia of over 250 mg/dL for two consecutive days in a row, fact which describes by itself the functionality of the circular device.

The animals with the flat or improved flattened device showed a more significant decrease of glucose levels to 150 mg/dL, fact which shows a 40% improvement in comparison with the circular device. This fact can be interpreted as a better performance of the flat or improved flattened device as a result of a higher number of surviving cells and functionally active.

In a preferred embodiment of this invention, instead of inserting various independent flat devices to obtain the number of cells required by each receptor the flat or improved flattened device is wider, with the plunger combed- shape one next to the other and/or one above the other in the central porous body, whose dimensions will be adjusted to the plungers width. Once the device has been covered by fibrocollagen with the neoformed vessels; the combed-shape plunger is replaced by the cell culture trays or the cells are implanted directly in the liquid medium into the immunologically privileged spaces left by the plungers. With this, the performance of the improved device is increased even more and facilitates the insertion of the culture trays, because instead of making various incisions depending on the number of devices inserted in the recipient organism, only one incision is made, locating the combed-shape plunger which is replaced by the cell culture trays. In the preferred embodiment four culture trays are used as it can be seen with more details in Figure 5.

It is herein stated that up to this date, the best known method by the applicant to implement said invention, is the one resulting from the description of this invention.

## Claims

1. A flat or improved flattened device to favor the implant of biological material implant wherein the device is composed of:
a. a central porous body, flat or flattened preferably rectangular or square;
b. sealing mechanisms in the corresponding ends of said porous body;
c. a plunger with integrated sealing element, with such geometry and dimensions that allow the plunger to be inserted tightly in the porous body. The plunger is made of a material that when it interacts with the tissues of a living organism tissues favors the formation of fibrocollagen on the surface and through the porous body;
d. one or various cell culture trays for depositing/culture cells in one or both (anterior and posterior) sides;
e. culture without tray in one or more fibrocollagen channels formed by the porous body.

2. An improved device to favor the implant of biological material in accordance with claim 1, wherein its novel flat shape decreases the inner dead space and increases the useful contact surface between the implanted cells and the neoformed fibrocollagen vessels.

3. An improved device to favor the implant of biological material in accordance with claim 1, wherein the central porous body is preferably integrated by a mesh with a shape and variable dimensions in accordance with the therapeutic applications and needs in terms of the number of cells to be implanted and the cell culture tray (s) to be inserted which function is to provide with a geometric and dimensional stability and support to the formation of fibrocollagen.

4. An improved device to favor the implant of biological material in accordance with claim 3, wherein the grid size is preferably between 40 and 150 mesh.

5. An improved device to favor the implant of biological material in accordance with claim 3, wherein he length of the porous body is preferably between 10 and 300 mm and the inner space in the porous body is preferaably between 0.1 to 250 millimeters.

6. An improved device to favor the implant of biological material in accordance with claim 3, wherein the porous body shows preferably at its ends sealing elements which are pressure-coupling elements with a latch as security element to allow the coupling of the assembly elements.

7. An improved device to favor the implant of biological material in accordance with claim 1, wherein the plunger has a shape and dimensions that allow its insertion in the porous body and its proximal end. The plunger is part of one of the assembly elements which allows its coupling to the sealing elements of the porous body and shows a wedge with a design such that holds the plunger and facilitates its withdrawal.

8. An improved device to favor the implant of biological material in accordance with claim 1, wherein the device has one or more of cell culture tray(s) culture that has one or both porous side(s) and abutments/supports in its ends, that together allows the *in vitro* culture and fixation implant on its sides of the cells to be implanted.

9. An improved device to favor the implant of biological material, in accordance with claim 8, wherein the cells culture tray(s) side(s) is one anterior and another posterior; preferably made of medical degree steel mesh or of any other material accepted for implants, with a porosity or surface that allows the cells to stick to both side(s) of the surfaces.

10. An improved device to favor the implant of biological material implant, in accordance with claim 8, wherein the cell culture tray(s) side (s) has in its ends assembly elements which allows its coupling to the porous body, by sealing the ends and leaving an isolated interior space.

11. An improved device to favor the implant of biological material, in accordance with claim 8, wherein the cell culture tray(s) in each of its ends has abutments/supports with a width enough that allows its coupling to the inner space of the porous body.

12. An improved device to favor the implant of biological material, in accordance with claim 8, wherein the abutments/supports of the tray(s) allows to fix it inside the porous body leaving in this way, a space between the side(s) of the tray(s) and the porous body enough in such a way that the seeded and implanted cells get in contact immediate and totally with the neoformed vessels.

13. An improved device to favor the implant of biological material, in accordance with claim 12, wherein the space between the culture tray(s) side(s) and the inner walls of the porous body is preferably between 0.1 mm to 3 mm.

14. An improved device to favor the implant of biological material, in accordance with claim 1, wherein the device is made of sterilizable materials.

15. A flat or improved flattened device to favor the implant of biological material in accordance with claim 1 wherein once it has been implanted in the organism it complies with the function of facilitating the formation or deposit of fibrocollagen and coating said deposit until a neovascularized reservoir of fibrocollagen is formed which allows to implant living cells into the reservoir of neovascularized fibrocollagen.

16. A flat or improved flattened device to favor the implant of biological material in accordance with claim 15 wherein cells are preferably cells producers of biological factors.

17. A flat or improved flattened device to favor the implant of biological material implant in accordance with claim 15 wherein those living cells are preferably Islets of Langerhans.

18. A flat or flattened improved device to favor the implant of biological material in accordance with claim 15, wherein the cells are primed cells against tumors.

19. A flat or improved flattened device to favor the implant of biological material implant wherein the flat or novel flattened design device together with the cell culture tray culture allows to enhance the survival rate of cells, fact which results in an improved performance in comparison with the circular devices.

20. A flat or improved flattened device to favor the implant of biological material wherein implant the device consists of:
a. flat or flattened central porous body preferably rectangular or square divided into various compartments;
b. sealing mechanisms in the corresponding ends of said porous body;
c. comb-shaped plunger with two or more arms, with integrated sealing mechanisms, with such geometry and dimensions that allow it to insert tightly inside the sectioned porous body, made of such material that when it interacts with tissues of living organism, improves the formation of fibrocollagen on the porous body surface;
d. trays to culture seed cells on the anterior and posterior sides in the same number of comb-shaped plungers.

21. An improved device to favor the implant of biological material, in accordance with claim 20, wherein it has a novel flat shape which decreases the dead space inside and increases the useful surface for the contact of implanted cells with the neoformed vessels of fibrocollagen.

22. An improved device to favor the implant of biological material, in accordance with claim 20, wherein the central porous body is preferentially composed of a flat shaped grid with variable dimensions according to the number of cells to be implanted, sectioned according to the number of cell culture trays to be inserted, whose function is to provide with geometric, and dimensional stability and to serve as a support for the formation of fibrocollagen.

23. An improved device to favor the implant of biological material, in accordance with claim 22, wherein the grid size is preferably of 40 to 150 mesh.

24. An improved device to favor the implant of biological material, in accordance with claim 22, wherein the length of the porous body is preferably of 10 to 300 mm and the interior space in the porous body is preferably of 0.1 to 250 mm.

25. An improved device to favor the implant of biological material, in accordance with claim 22, wherein the porous body has in its ends, the assembly mechanisms that are pressure coupling mechanisms or with a latch as security element in order to allow the coupling of the assembly elements.

26. An improved device to favor the implant of biological material in accordance with claim 20, wherein the plunger i comb-shaped with two or more trays, in accordance with the number of sections of the porous body, with such shape and dimensions that allows the insertion in the porous body. At its proximal end, the device is part of one of the assembly elements that allows the coupling to the assembly elements of the porous body and show a wedge with such design that holds the plunger facilitates its withdrawal.

27. An improved device to favor the implant of biological material, in accordance with the claim 20, wherein such device has two or more cell culture trays, which shows one and/or two porous sides and abutments/supports in the ends, together allow the in vitro culturing and fixation of the cells to be implanted in its sides.

28. An improved device to favor the implant of biological material in accordance with claim 27, wherein the cell culture trays have two sides, one anterior and the other posterior. Preferably the device is made of medical degree steel mesh or of any other material accepted for the implant, with such porosity that allows the cells to be cultured to adhere on both sides of the surface.

29. An improved device to favor the implant of biological material in accordance with claim 27, wherein the cell culture tray(s) has in each of its ends, abutments/supports with such dimensions and shapes that allow its coupling to the inner space of the porous body.

30. An improved device to favor the implant of biological material in accordance with claim 27, wherein the abutments/supports of the tray(s) allow to fix it inside the porous body, thus leaving a space enough between the tray(s) sides and the porous body in such a way that the seeded and implanted cells can enter immediately and totally in contact with the neoformed vessels.

31. An improved device to favor the implant of biological material in accordance with claim 27, wherein the space between the cell culture tray and the inner walls of the porous body left by the abutments/supports is preferably between 0.1 mm and 3 mm.

32. An improved device to favor the implant of biological material in accordance with claim 27, wherein the ends of the tray(s) have assembly elements that allow its coupling to the porous body, sealing in this way its ends and leaving an isolated inner space.

33. An improved device to favor the implant of biological material in accordance with claim 20 wherein the device is made of sterilizable materials.

34. A flat or improved flattened device to favor the implant of biological material implant in accordance with claim 20 wherein once it has been implanted in the organism it complies with its function of facilitating the formation or deposit of fibrocollagen and coating said deposit until a neovascularized reservoir of fibrocollagen is formed to allows to implant living cells into the reservoir of neovascularized fibrocollagen.

35. A flat or improved flattened device to favor the implant biological material in accordance with claim 34, wherein the cells are preferably producers of biological factors.

36. A flat or improved flattened device to favor the implant of biological material in accordance with claim 34, wherein the living cells are preferably Islets of Langerhans.

37. A flat or improved flattened device to favor the implant of biological material in accordance with claim 34, characterized because the cells are primed cells against tumors.

38. A flat or improved flattened device to implant biological material, wherein the device has a flat/flattened novel device with a comb-shaped plunger and various cell culture trays, which allow to obtain improvements on the cells survival, thus resulting in an improved performance when compared with circular devices.
